# EUROPEAN PATENT APPLICATION

(11) **EP 4 599 697 A1**
(43) Date of publication of application: **13.08.2025**
(21) Application number: 23874960.0
(22) Date of filing: 06.10.2023
(51) Int. Cl.: A23L 33/20, A61K 31/197, A61K 31/683, A61K 36/02, A61P 1/16, A61P 3/00, A61P 3/04, A61P 3/06

(54) **ACTION FOR SUPPRESSING ANY INCREASE IN BODY WEIGHT BY AQUEOUS EXTRACT OF NANNOCHLOROPSIS ALGAE**

(30) Priority: 07.10.2022 JP 2022162515
(71) Applicant: Phytolipid Technologies Co., Ltd., Yokohama-shi, Kanagawa 226-8510 (JP); KANAGAWA INSTITUTE OF INDUSTRIAL SCIENCE AND TECHNOLOGY, Ebina-shi Kanagawa 243-0435 (JP)
(72) Inventor: YANO, Atsushi, Yokohama-shi, Kanagawa 245-0053 (JP); UMEZU, Akira, Yokohama-shi, Kanagawa 245-0053 (JP); MITOME, Kanoko, Yokohama-shi, Kanagawa 245-0053 (JP); SASAKI, Yuko, Yokohama-shi, Kanagawa 226-8510 (JP); KAMEI, Asuka, Ebina-shi, Kanagawa 243-0435 (JP); SHIMADA, Kosuke, Ebina-shi, Kanagawa 243-0435 (JP); SHINOZAKI, Fumika, Ebina-shi, Kanagawa 243-0435 (JP); SHIMOJIMA, Mie, Tokyo 152-8550 (JP); OHTA, Hiroyuki, Tokyo 152-8550 (JP); IWAI, Masako, Tokyo 152-8550 (JP); IHARA, Yuta, Tokyo 152-8550 (JP)
(74) Representative: Eisenführ Speiser
(86) International application number: PCT/JP2023/036520
(87) International publication number: WO 2024/075835

(57) **Abstract**

The present invention provides a novel use of Nannochloropsis algae. Specifically, a water extract of Nannochloropsis algae is used for suppressing a body weight increase or inhibiting cholesterol synthesis promotion.

## Description

### TECHNICAL FIELD

The present invention relates to a body weight increase suppressing agent, food/drink for suppressing body weight increase, a cholesterol synthesis promotion inhibitor, food/drink for inhibiting cholesterol synthesis promotion, and a pharmaceutical composition for treating a disease associated with cholesterol synthesis promotion, which are characterized by containing a water extract of Nannochloropsis algae as an active ingredient.

### BACKGROUND ART

Extracts of Nannochloropsis algae are known to have various biological activities.

Patent Literature 1 describes that an aqueous ethanol extract of Nannochloropsis algae has an anti-inflammatory activity.

Patent Literature 2 describes that an extract of Nannochloropsis algae increases paraoxonase 1 (PON1) activity which suppresses the oxidation of low-density lipoprotein (LDL).

Non-Patent Literature 1 describes that a substance isolated from Nannochloropsis algae improves the function of high-density lipoprotein (HDL).

### CITATION LIST

### PATENT LITERATURE

Patent Literature 1: Japanese Unexamined Patent Application Publication No. 2015-174850
Patent Literature 2: WO2019/026067

### NON-PATENT LITERATURE

Non-Patent Literature 1: BioFactors. 2020; 46: 146-157

### SUMMARY OF INVENTION

### TECHNICAL PROBLEM

The present inventors focused on Nannochloropsis algae as a raw material for e.g. health food, and to provide novel uses thereof was set as a subject of the present invention.

### SOLUTION TO PROBLEM

As a result of diligent investigations on this subject, the present inventors found that a water extract of Nannochloropsis algae suppressed body weight increase and further inhibited cholesterol synthesis promotion. The present invention has been made based on these findings.

That is, the present invention relates to [1] to [25] described below.
[1] A body weight increase suppressing agent, comprising a water extract of Nannochloropsis algae as an active ingredient.
[2] The suppressing agent according to [1], wherein the water extract comprises one or more compounds selected from [A group] described below:
[A group]
15-HEPE,
a monoacylglyceryltrimethylhomoserine represented by the general formula (I): 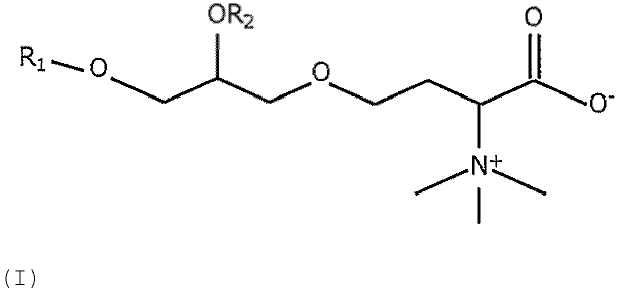 wherein R₁ and R₂ represent combination A, B, E, or F described below:

| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |

and
a lysophosphatidylcholine represented by the general formula (II) : 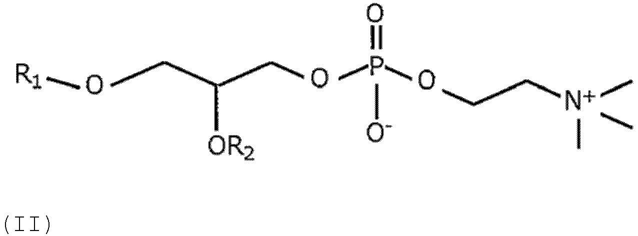 wherein R₁ and R₂ represent combination A or B described below.

| Combination | R₁ | R₂ |
|---|---|---|
| A | HEPE residue | Hydrogen |
| B | Hydrogen | HEPE residue |

[3] The suppressing agent according to [2], wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:

| Combination | R₁ | R₂ |
|---|---|---|
| C | Hydroxy eicosatetraenoic acid (HETE) residue | Hydrogen |
| D | Hydrogen | HETE residue |

and
a lysophosphatidylcholine represented by the general formula (II),
wherein R₁ and R₂ represent combination C or D.

| Combination | R₁ | R₂ |
|---|---|---|
| C | Hydroperoxy eicosapentaenoic acid (HpEPE) residue | Hydrogen |
| D | Hydrogen | HpEPE residue |

[4] The suppressing agent according to [1], wherein the Nannochloropsis algae are Nannochloropsis oceanica.
[5] The suppressing agent according to [1], which suppresses body weight increase associated with high-fat diet intake.
[6] Food/drink for suppressing body weight increase, comprising a water extract of Nannochloropsis algae as an active ingredient.
[7] The food/drink according to [6], wherein the water extract comprises one or more compounds selected from [A group] described below:
[A group]
15-HEPE,
a monoacylglyceryltrimethylhomoserine represented by the general formula (I): 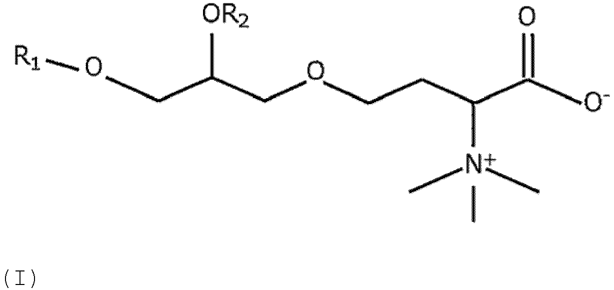 wherein R₁ and R₂ represent combination A, B, E, or F described below:

| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |

and
a lysophosphatidylcholine represented by the general formula (II) : 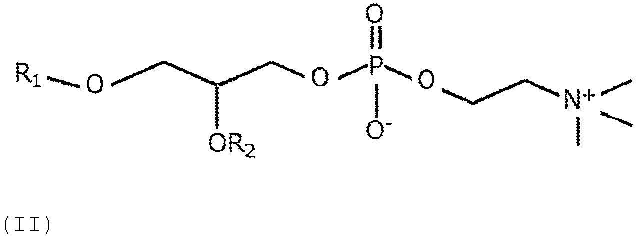 wherein R₁ and R₂ represent combination A or B described below.

| Combination | R₁ | R₂ |
|---|---|---|
| A | HEPE residue | Hydrogen |
| B | Hydrogen | HEPE residue |

[8] The food/drink according to [7], wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:

| Combination | R₁ | R₂ |
|---|---|---|
| C | Hydroxy eicosatetraenoic acid (HETE) residue | Hydrogen |
| D | Hydrogen | HETE residue |

and
a lysophosphatidylcholine represented by the general formula (II),
wherein R₁ and R₂ represent combination C or D.

| Combination | R₁ | R₂ |
|---|---|---|
| C | Hydroperoxy eicosapentaenoic acid (HpEPE) residue | Hydrogen |
| D | Hydrogen | HpEPE residue |

[9] The food/drink according to [6], wherein the Nannochloropsis algae are Nannochloropsis oceanica.
[10] The food/drink described in [6] above, which suppresses body weight increase associated with high-fat diet intake.
[11] A cholesterol synthesis promotion inhibitor, comprising a water extract of Nannochloropsis algae as an active ingredient.
[12] The inhibitor according to [11], wherein the water extract comprises one or more compounds selected from [A group] described below:
[A group]
15-HEPE,
a monoacylglyceryltrimethylhomoserine represented by the general formula (I): 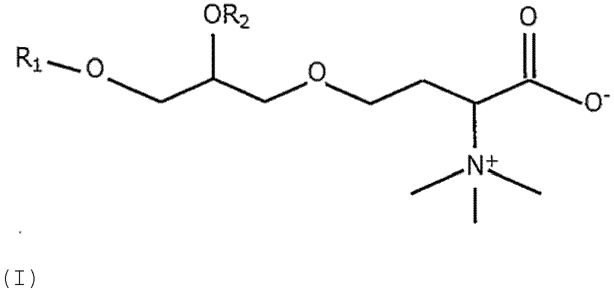 wherein R₁ and R₂ represent combination A, B, E, or F described below:

| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |

and
a lysophosphatidylcholine represented by the general formula (II) : 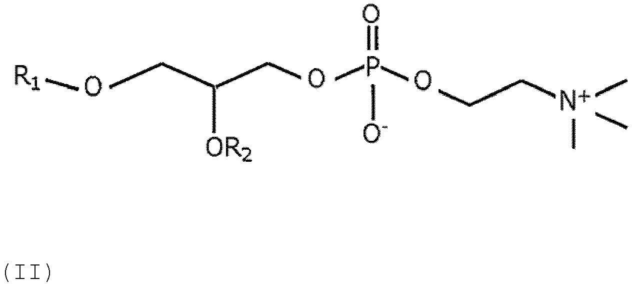 wherein R₁ and R₂ represent combination A or B described below.

| Combination | R₁ | R₂ |
|---|---|---|
| A | HEPE residue | Hydrogen |
| B | Hydrogen | HEPE residue |

[13] The inhibitor according to [12], wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:

| Combination | R₁ | R₂ |
|---|---|---|
| C | Hydroxy eicosatetraenoic acid (HETE) residue | Hydrogen |
| D | Hydrogen | HETE residue |

and
a lysophosphatidylcholine represented by the general formula (II),
wherein R₁ and R₂ represent combination C or D.

| Combination | R₁ | R₂ |
|---|---|---|
| C | Hydroperoxy eicosapentaenoic acid (HpEPE) residue | Hydrogen |
| D | Hydrogen | HpEPE residue |

[14] The inhibitor according to [11], wherein the Nannochloropsis algae are Nannochloropsis oceanica.
[15] The inhibitor according to [11], which inhibits cholesterol synthesis promotion associated with high-fat diet intake.
[16] Food/drink for inhibiting cholesterol synthesis promotion, comprising a water extract of Nannochloropsis algae as an active ingredient.
[17] The food/drink according to [16], wherein the water extract comprises one or more compounds selected from [A group] described below:
[A group]
15-HEPE,
a monoacylglyceryltrimethylhomoserine represented by the general formula (I): 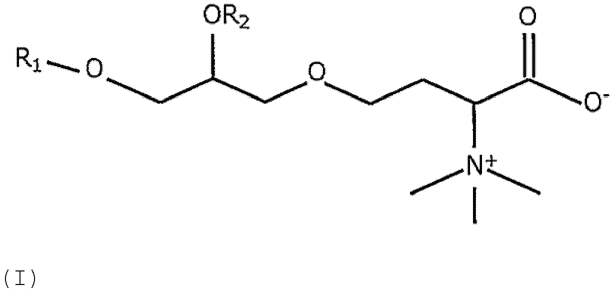 wherein R₁ and R₂ represent combination A, B, E, or F described below:

| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |

and
a lysophosphatidylcholine represented by the general formula (II) : 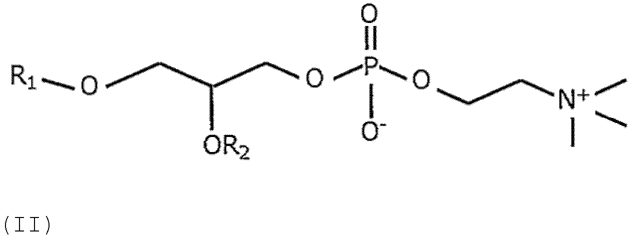 wherein R₁ and R₂ represent combination A or B described below.

| Combination | R₁ | R₂ |
|---|---|---|
| A | HEPE residue | Hydrogen |
| B | Hydrogen | HEPE residue |

[18] The food/drink according to [17], wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:

| Combination | R₁ | R₂ |
|---|---|---|
| C | Hydroxy eicosatetraenoic acid (HETE) residue | Hydrogen |
| D | Hydrogen | HETE residue |

and
a lysophosphatidylcholine represented by the general formula (II),
wherein R₁ and R₂ represent combination C or D.

| Combination | R₁ | R₂ |
|---|---|---|
| C | Hydroperoxy eicosapentaenoic acid (HpEPE) residue | Hydrogen |
| D | Hydrogen | HpEPE residue |

[19] The food/drink according to [16], wherein the Nannochloropsis algae are Nannochloropsis oceanica.
[20] The food/drink described in [16] above, which inhibits cholesterol synthesis promotion associated with high-fat diet intake.
[21] A pharmaceutical composition for treating a disease associated with cholesterol synthesis promotion, comprising a water extract of Nannochloropsis algae as an active ingredient.
[22] The pharmaceutical composition according to [21], wherein the water extract comprises one or more compounds selected from [A group] described below:
[A group]
15-HEPE,
a monoacylglyceryltrimethylhomoserine represented by the general formula (I): 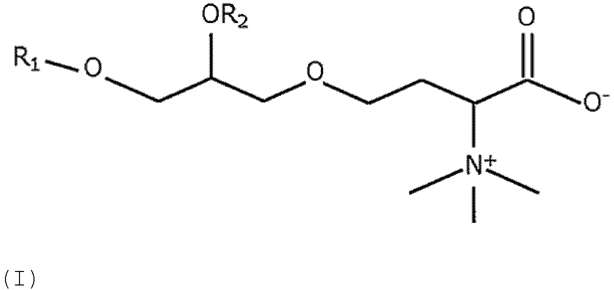 wherein R₁ and R₂ represent combination A, B, E, or F described below:

| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |

and
a lysophosphatidylcholine represented by the general formula (II) : 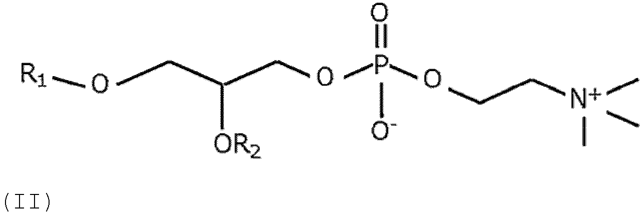 wherein R₁ and R₂ represent combination A or B described below.

| Combination | R₁ | R₂ |
|---|---|---|
| A | HEPE residue | Hydrogen |
| B | Hydrogen | HEPE residue |

[23] The pharmaceutical composition according to [22], wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:

| Combination | R₁ | R₂ |
|---|---|---|
| C | Hydroxy eicosatetraenoic acid (HETE) residue | Hydrogen |
| D | Hydrogen | HETE residue |

and
a lysophosphatidylcholine represented by the general formula (II),
wherein R₁ and R₂ represent combination C or D.

| Combination | R₁ | R₂ |
|---|---|---|
| C | Hydroperoxy eicosapentaenoic acid (HpEPE) residue | Hydrogen |
| D | Hydrogen | HpEPE residue |

[24] The pharmaceutical composition according to [21], wherein the Nannochloropsis algae are Nannochloropsis oceanica.
[25] The pharmaceutical composition according to [21], wherein the disease associated with cholesterol synthesis promotion is selected from the group consisting of non-alcoholic fatty liver disease (NAFLD), non-alcoholic steatohepatitis (NASH), dyslipidemia, and metabolic syndrome.

### ADVANTAGEOUS EFFECT OF INVENTION

As described in Examples below, a water extract of Nannochloropsis algae suppresses body weight increase and further inhibits cholesterol synthesis promotion. The present invention using this water extract as an active ingredient is useful for e.g. treating body weight increase and diseases associated with cholesterol synthesis promotion.

### BRIEF DESCRIPTION OF DRAWING

Fig. 1 is a box plot showing the distribution of the amount of body weight increase in individuals of each test group; o: value of each individual, x: average value, LFD: Test group 1, HFD: Test group 2, HFD + HL: Test group 3, and HFD + HH: Test group 4.
Fig. 2 is a box plot showing the distribution of the rate of body weight increase (when the base date is considered 1) in individuals of each test group; o: value of each individual, x: average value, LFD: Test group 1, HFD: Test group 2, HFD + HL: Test group 3, HFD + HH: Test group 4.

### DESCRIPTION OF EMBODIMENT

### [Nannochloropsis algae]

Nannochloropsis is single-celled algae belonging to the phylum Heterokontophyta, class Eustigmatophyceae.

Nannochloropsis algae can be used without particular restrictions, and specific examples thereof include Nannochloropsis oceanica, Nannochloropsis oculata, Nannochloropsis gaditana, Nannochloropsis salina, Nannochloropsis atomus, Nannochloropsis maculata, Nannochloropsis granulata, Nannochloropsis sp. and the like. Among these, Nannochloropsis oceanica and Nannochloropsis gaditana are preferably used and Nannochloropsis oceanica is particularly preferably used.

Nannochloropsis oceanica include NIES-2145 strain deposited in Microbial Culture Collection at the National Institute for Environmental Studies, National Research and Development Agency (NIES Collection), and CCMP-1779 strain deposited in NCMA (The National Center for Marine Algae and Microbiota) (USA), and NIES-2145 strain is preferred.

Nannochloropsis algae can be available from Microorganism Depositary and the market, and may be also isolated from nature.

The water extract of the present invention may be prepared from one or more Nannochloropsis algae.

### [Water extract of Nannochloropsis algae]

The water extract of Nannochloropsis algae (hereinafter referred to as algal water extract) can be used in the present invention without particular restrictions.

In a preferred aspect, the algal water extract comprises one or more of the following compounds (1) to (7).

(1) A monoacylglyceryltrimethylhomoserine (MGTS) represented by the general formula (I): 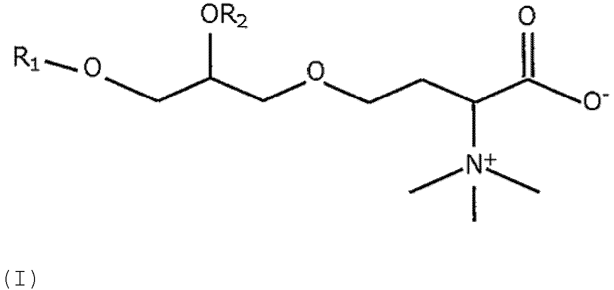 wherein R₁ and R₂ represent any one of combinations A to F described below.

| Combination | R₁ | R₂ |
|---|---|---|
| A | Hydroxy eicosapentaenoic acid (HEPE) residue | Hydrogen |
| B | Hydrogen | HEPE residue |
| C | Hydroxy eicosatetraenoic acid (HETE) residue | Hydrogen |
| D | Hydrogen | HETE residue |
| E | Eicosapentaenoic acid (EPA) residue | Hydrogen |
| F | Hydrogen | EPA residue |

The HEPE residue means a residue obtained by removing the hydroxy group from the carboxy group in HEPE.

Examples of the HEPE include 15-hydroxy eicosapentaenoic acid (15-HEPE), 8-HEPE, 11-HEPE, and 18-HEPE.

The compound of the general formula (I) (wherein R₁ and R₂ represent combination A) is also referred to as 1-(hydroxyeicosapentaenoyl)-sn-glycero-3-O-4'-(N,N,N-trimethyl)-homoserine or MGTS-1-HEPE.

The compound of the general formula (I) (wherein R₁ and R₂ represent combination B) is also referred to as 2-(hydroxyeicosapentaenoyl)-sn-glycero-3-O-4'-(N,N,N-trimethyl)-homoserine or MGTS-2-HEPE.

The HETE residue means a residue obtained by removing the hydroxy group from the carboxy group in HETE.

Examples of the HETE include 15-hydroxy eicosatetraenoic acid (15-HETE) and 11-HETE.

The compound of the general formula (I) (wherein R₁ and R₂ represent combination C) is also referred to as 1-(hydroxyeicosatetraenoyl)-sn-glycero-3-O-4'-(N,N,N-trimethyl)-homoserine or MGTS-1-HETE.

The compound of the general formula (I) (wherein R₁ and R₂ represent combination D) is also referred to as 2-(hydroxyeicosatetraenoyl)-sn-glycero-3-O-4'-(N,N,N-trimethyl)-homoserine or MGTS-2-HETE.

The EPA residue means a residue obtained by removing the hydroxy group from the carboxy group in EPA.

The compound of the general formula (I) (wherein R₁ and R₂ represent combination E) is referred to as 1-(eicosapentaenoyl)-sn-glycero-3-O-4'-(N,N,N-trimethyl)-homoserine or MGTS-1-EPA.

The compound of the general formula (I) (wherein R₁ and R₂ represent combination F) is also referred to as 2-(eicosapentaenoyl)-sn-glycero-3-O-4'-(N,N,N-trimethyl)-homoserine or MGTS-2-EPA.

(2) A lysophosphatidylcholine (LPC) represented by the general formula (II): 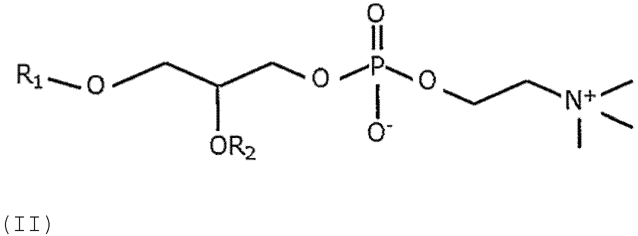 wherein R₁ and R₂ represent any one of combinations A to D described below.

| Combination | R₁ | R₂ |
|---|---|---|
| A | HEPE residue | Hydrogen |
| B | Hydrogen | HEPE residue |
| C | Hydroperoxy eicosapentaenoic acid (HpEPE) residue | Hydrogen |
| D | Hydrogen | HpEPE residue |

The HEPE residue means a residue obtained by removing the hydroxy group from the carboxy group in HEPE.

Examples of the HEPE include 15-hydroxy eicosapentaenoic acid (15-HEPE), 8-HEPE, 11-HEPE, and 18-HEPE.

The compound of the general formula (II) (wherein R₁ and R₂ represent combination A) is also referred to as 1-(hydroxyeicosapentaenoyl)-sn-glycero-3-phosphocholine or LPC-1-HEPE.

The compound of the general formula (II) (wherein R₁ and R₂ represent combination B) is also referred to as 2-(hydroxyeicosapentaenoyl)-sn-glycero-3-phosphocholine or LPC-2-HEPE.

The HpEPE residue means a residue obtained by removing the hydroxy group from the carboxy group in HpEPE.

Examples of the HpEPE include 15-hydroperoxy eicosapentaenoic acid (15-HpEPE), 8-HpEPE, 11-HpEPE, and 18-HpEPE.

The compound of the general formula (II) (wherein R₁ and R₂ represent combination C) is also referred to as 1-(hydroperoxyeicosapentaenoyl)-sn-glycero-3-phosphocholine or LPC-1-HpEPE.

The compound of the general formula (II) (wherein R₁ and R₂ represent combination D) is also referred to as 2-(hydroperoxyeicosapentaenoyl)-sn-glycero-3-phosphocholine or LPC-2-HpEPE.

### (3) 15-Hydroxy eicosapentaenoic acid (15-HEPE)

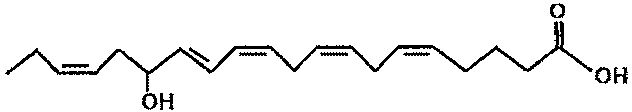

### (4) 15-Hydroxy eicosatetraenoic acid (15-HETE)

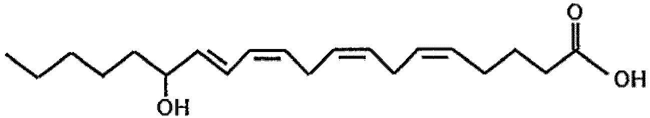

### (5) 15-Hydroxy eicosatrienoic acid (15-HETrE)

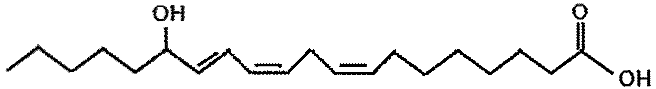

### (6) 13-Hydroxy octadecatrienoic acid (13-HOTrE)

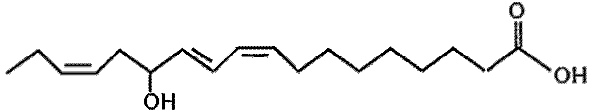

### (7) 13-Hydroxy octadecadienoic acid (13-HODE)

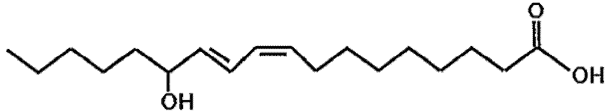

In a preferred aspect, the algal water extract may comprise one or more, preferably two or more, and further preferably 4 or more compounds (A group compounds) selected from A group described below:
[A group]
15-HEPE,
a compound of the general formula (I) (wherein R₁ and R₂ represent combination A or B),
a compound of the general formula (I) (wherein R₁ and R₂ represent combination E or F), and
a compound of the general formula (II) (wherein R₁ and R₂ represent combination A or B).

The algal water extract may comprise one or more, preferably 3 or more, and further preferably 6 or more compounds selected from B group described below.

[B group]
13-HOTrE,
13-HODE,
15-HETrE,
15-HETE,
a compound of the general formula (I) (wherein R₁ and R₂ represent combination C or D), and
a compound of the general formula (II) (wherein R₁ and R₂ represent combination C or D).

### [Method for producing algal water extract]

The method for producing the algal water extract is not particularly restricted, and the method preferably includes 3 steps, "culturing of an alga," "crushing of the alga," and "water extraction of homogenized algal material", and further preferably includes [column purification of a water extract] additionally.

### [Culturing of alga]

Nannochloropsis algae can be cultured according to a known method.

In addition to ESM medium used in Examples described below, media provided in "2. Media for marine and brackish water microalgae" in NIES collection homepage ([online], [searched on September 21, 2022], Internet, https://mcc.nies.go.jp/02medium.html), for example, can be used as a medium.

Culture conditions can be properly set depending on algal types and culturing scale, and Nannochloropsis oceanica is preferably cultured with shaking or aerobically cultured at a temperature of 20 to 25°C under white light irradiation or natural light (preferably photon flux density: 17 to 300 µmol/m²/s).

### [Crushing of alga]

An active ingredient is believed to be accumulated in algal cells. In order to increase the yield of the active ingredient, it is preferred to crush algal cells before water extraction in the present invention.

The crushing procedure is not particularly restricted as long as algal cells can be broken. For example, algal cells recovered from a cultured fluid are suspended in a liquid medium and the suspension is subjected to crushing means to obtain homogenized algal material.

Examples of the liquid medium include water, alcohol, or a mixed liquid of water and alcohol, and water (particularly pure water) is preferably used from the viewpoint of safety in scaling up. Alcohol is methanol, ethanol, or the like, and methanol is preferably used. The amount of alcohol contained in the mixed liquid of water and alcohol is 20 to 80 mass% and preferably 40 to 60 mass% with respect to the total mass of the mixed liquid. To the liquid medium, 2-morpholinoethanesulfonic acid may be added for the purpose of adjusting pH.

Examples of the crushing means include a bead type homogenizer, a pressure type homogenizer, an ultrasonic homogenizer and the like.

### [Water extraction of homogenized algal material]

The procedure of water extraction is not particularly restricted, and it is only needed to mix the homogenized algal material and water. It is preferred to add water to the homogenized algal material; however, the homogenized algal material may be also added to water.

The homogenized algal material is preferably gelled using a gelling agent before water extraction.

As water as an extraction solvent, tap water, ion-exchanged water, pure water, ultrapure water, distilled water and the like can be used without particular restrictions, and pure water or ultrapure water is preferably used from the viewpoint of the purity of an extract. Pure water with an electric conductivity of 1 µS/cm or less is preferably used. Ultrapure water with an electric conductivity of 0.0549 µS/cm or less and a TOC of 5 ppb or less is preferably used. Pure water and ultrapure water can be produced using a commercially available production device (e.g. Milli-Q (registered trademark)).

The amount of water used is not particularly restricted, and water is used in a volume corresponding to a mass of 2 to 1000 times, preferably 5 to 100 times, and more preferably 10 to 80 times larger than the wet mass (wet weight) of an algal body used for crushing.

Extraction is preferably continuous extraction in which water (extraction solvent) is supplied while collecting an extract.

Extraction is preferably carried out with stirring from the viewpoint of extraction velocity.

The extraction temperature is not particularly restricted, and is preferably 4 to 25°C from the viewpoint of suppressing the decomposition of an active ingredient.

The extraction time is not particularly restricted, and is, for example, 0.5 to 36 hours.

### [Column purification of algal water extract]

In order to increase the purity of the algal water extract, the water extract is preferably subjected to column purification.

For column purification, a column purification technique using hydrophobic interactions can be used. A solid phase column (e.g. product name: STRATA-X, supplier: SHIMADZU GLC) including a polymer (e.g. styrene/divinylbenzene polymer) into which a functional group (e.g. N-vinylpyrrolidone) effective to retain a polar compound is introduced and a solid phase column in which a synthetic adsorbent (product name: SP850, supplier: Mitsubishi Chemical Corporation) including a styrene polymer having a porous structure is packed, for example, can be suitably used.

Column purification may be carried out several times.

### [Body weight increase suppressing agent]

One aspect of the present invention is a body weight increase suppressing agent, comprising a water extract of Nannochloropsis algae (hereinafter referred to as "algal water extract") as an active ingredient.

The cause of body weight increase is not particularly restricted, but the present invention can be suitably used against body weight increase associated with high-fat diet intake. Examples of the high-fat diet include a meal comprising 20 to 70% of fat in terms of an energy ratio.

"Containing as an active ingredient" means that the body weight increase suppressing agent comprises the algal water extract in an amount sufficient to display a desired suppressing effect (i.e. effective amount).

The algal water extracts can be used individually or a plurality of the algal water extracts can be used in combination.

The amount of the algal water extract contained can be properly set considering e.g. the form of the agent (such as food/drink or a pharmaceutical product). In the case of liquid food for human, for example, the amount of the "A group compound" contained as an active ingredient is preferably 0.001 to 8.35 mg/mL, and further preferably 0.005 to 3.34 mg/mL with respect to the total volume of the liquid food.

The body weight increase suppressing agent may comprise one or more optional components without impairing the action of an active ingredient. The optional components can be properly selected depending on **e.g.** the form of the agent.

The optional components may be additives for food/drink or medicine.

The optional components are known substances, and are easily available in the market or can be prepared.

The optional components can be used individually or a plurality of the optional components can be used in combination.

The amount of an optional component contained can be properly set depending on e.g. the purpose of mixing the component.

The body weight increase suppressing agent can be applied to a wide range of animal species without particular restrictions. The subject to be applied is preferably a mammal (human and non-human mammals (e.g. bovine and equine)), and more preferably human. In addition, the sex and age of the subject to be applied do not matter.

The intake amount of the body weight increase suppressing agent can be properly set depending on e.g. the age and body weight of the subject to be applied, the administration route, or the frequency of administration. In the case of oral intake in human, for example, the intake amount of the "A group compound" is preferably 0.00162 to 0.815 mg/kg body weight/day, and further preferably 0.004 to 0.362 mg/kg body weight/day.

The intake interval can be properly set depending on e.g. the intake amount, and it may be once a day or a few times a day.

### [Method for preparing body weight increase suppressing agent]

The body weight increase suppressing agent can be prepared by mixing an active ingredient (algal water extract) and an optional component (e.g. additives for food/drink or medicine).

### [Use form of body weight increase suppressing agent]

The body weight increase suppressing agent can be used as food/drink or a pharmaceutical product.

### [Food/drink]

The form of the food/drink is not particularly restricted as long as it can be orally taken. Specific examples thereof include liquid drink and jelly drink.

The food/drink is e.g. health food, functional food, nutritional supplementary food, specified health food, medical food, or food/drink with reduction of disease risk claims.

The food/drink may include a food additive as an optional component.

An additive for liquid drink is e.g. a pH adjuster, an emulsifier, a stabilizer, a flavoring, or a sweetener. An additive for jelly drink is e.g. gelatin, a food dye, or a thickening polysaccharide.

### [Pharmaceutical product]

The dosage form of the pharmaceutical product is preferably a form that can be administered orally or through an intravenous drip. Specific examples thereof include liquid medicines, capsules, and powder.

The pharmaceutical product may include a pharmaceutical additive as an optional component. Examples of the pharmaceutical additive include an excipient, a stabilizer, a preservative, a wetting agent, an emulsifier, a lubricant, a sweetener, a coloring agent, a flavoring, a buffering agent, an antioxidant, and a pH adjuster.

### [Cholesterol synthesis promotion inhibitor]

One aspect of the present invention is a cholesterol synthesis promotion inhibitor, comprising a water extract of Nannochloropsis algae (hereinafter referred to as "algal water extract") as an active ingredient.

The cause of cholesterol synthesis promotion is not particularly restricted, but the present invention can be suitably used against cholesterol synthesis promotion associated with high-fat diet intake. Examples of the high-fat diet include a meal comprising 20 to 70% of fat in terms of an energy ratio.

"Inhibiting cholesterol synthesis promotion" means to inhibit the expression of genes involved in cholesterol synthesis promotion in the cholesterol synthesis system. Examples of such genes in human include 11 genes described below.

| Gene name |
|---|
| 3-hydroxy-3-methylglutaryl-Coenzyme A synthase 1 |
| 3-hydroxy-3-methylglutaryl-Coenzyme A reductase |
| Mevalonate (diphospho) decarboxylase |
| Farnesyl diphosphate farnesyl transferase 1 |
| Squalene epoxidase |
| Lanosterol synthase |
| Cytochrome P450 family 51 subfamily A member 1 |
| Methylsterol monoxygenase 1 |
| NAD(P) dependent steroid dehydrogenase-like |
| Hydroxysteroid (17-beta) dehydrogenase 7 |
| 7-Dehydrocholesterol reductase |

The algal water extract inhibits the expression of one or more of the above 11 genes, more preferably 5 or more of the genes, and further preferably all the 11 genes.

"Comprising as an active ingredient" means that the cholesterol synthesis promotion inhibitor comprises the algal water extract in an amount sufficient to display a desired inhibition effect (i.e. effective amount).

The algal water extracts may be used individually or a plurality of the algal water extracts may be used in combination.

The amount of the algal water extract contained can be properly set considering e.g. the form of the inhibitor (such as food/drink or a pharmaceutical product). In the case of liquid food for human, for example, the amount of the "A group compound" contained as an active ingredient is preferably 0.001 to 8.35 mg/mL, and further preferably 0.005 to 3.34 mg/mL with respect to the total volume of the liquid food.

The cholesterol synthesis promotion inhibitor may comprise one or more optional components without impairing the action of an active ingredient. The optional components can be properly selected depending on e.g. the form of the inhibitor.

The optional components may be additives for food/drink or medicine.

The optional components are known substances, and are easily available in the market or can be prepared.

The optional components may be used individually or a plurality of the optional components may be used in combination.

The amount of an optional component contained can be properly set depending on e.g. the purpose of mixing the component.

The cholesterol synthesis promotion inhibitor can be applied to a wide range of animal species having the cholesterol synthesis system without particular restrictions. The subject to be applied is preferably a mammal (human and non-human mammals (e.g. bovine and equine)), and more preferably human. In addition, the sex and age of the subject to be applied do not matter.

The intake amount of the cholesterol synthesis promotion inhibitor can be properly set depending on e.g. the age and body weight of the subject to be applied, the administration route, or the frequency of administration. In the case of oral intake in human, for example, the intake amount of the "A group compound" is preferably 0.00162 to 0.815 mg/kg body weight/day, and further preferably 0.004 to 0.362 mg/kg body weight/day.

The intake interval can be properly set depending on e.g. the intake amount, and it can be once a day or a few times a day.

### [Method for preparing cholesterol synthesis promotion inhibitor]

The cholesterol synthesis promotion inhibitor can be prepared by mixing an active ingredient (algal water extract) and an optional component (e.g. an additive for food/drink or medicine) .

### [Use form of cholesterol synthesis promotion inhibitor]

The body weight increase inhibitor can be used as food/drink or a pharmaceutical product.

### [Food/drink]

The form of food/drink is not particularly restricted as long as it can be orally taken. Specific examples thereof include liquid drink and jelly drink.

The food and drink is e.g. health food, functional food, nutritional supplementary food, specified health food, medical food, or food/drink with reduction of disease risk claims.

The food/drink may include a food additive as an optional component.

An additive for liquid drink is e.g. a pH adjuster, an emulsifier, a stabilizer, a flavoring and a sweetener. An additive for jelly drink is e.g. gelatin, a food dye, or a thickening polysaccharide.

### [Pharmaceutical product]

The cholesterol synthesis promotion inhibitor as a pharmaceutical product can be used to treat a "disease associated with cholesterol synthesis promotion." Examples of the "disease associated with cholesterol synthesis promotion" include non-alcoholic fatty liver disease (NAFLD), non-alcoholic steatohepatitis (NASH), dyslipidemia, metabolic syndrome, and the like.

The dosage form of the pharmaceutical product is preferably a form that can be administered orally or through an intravenous drip. Specific examples thereof include liquid medicines, capsules, and powder.

The pharmaceutical product (pharmaceutical composition) may comprise a pharmaceutical additive as an optional component. Examples of the pharmaceutical additive include an excipient, a stabilizer, a preservative, a wetting agent, an emulsifier, a lubricant, a sweetener, a coloring agent, a flavoring, a buffering agent, an antioxidant, and a pH adjuster.

### EXAMPLES

The present invention will now be described in more detail by way of examples. It should be noted, however, that the present invention is not limited thereto.

### [Culturing of Nannochloropsis algae]

Nannochloropsis oceanica NIES-2145 strain, provided from Microbial Culture Collection at the National Institute for Environmental Studies, National Research and Development Agency, was used.

As a culturing medium, ESM medium having the following composition was used.

| Component | Concentration |
|---|---|
| KNO₃ | 2.5 g/L |
| Na₂HPO₄ | 0.25 g/L |
| Vitamin B12 | 2.5 µg/L |
| Biotin | 2.5 µg/L |
| Thiamin | 0.5 µg/L |
| Fe-EDTA | 0.075 g/L |
| ZnSO₄•7H₂O | 1.11 mg/L |
| CuSO₄•5H₂O | 0.395 mg/L |
| MoO₃ | 0.075 mg/L |
| H₃BO₃ | 14.3 mg/L |
| MnCl₂•4H₂O | 9.05 mg/L |
| MARINE ART SF-1 | 38.3 g/L |
| Water | Amount to obtain 1 L of the total medium amount |

To a 11 L clear container with 9 L of ESM medium, 1 L of NIES-2145 strain precultured fluid (the number of cells: 1 × 10⁹ cells/mL) was inoculated (the number of cells in the medium after inoculation: 1 x 10⁸ cells/mL). Cultivation was performed under white light irradiation (light source: white LED, photon flux density: 300 µmol/m²/s) at a liquid temperature of 20°C while blowing (flow rate: 3 L/min) air (CO₂ concentration: 2%) into the clear container.

### [Crushing of alga]

About 70 g (wet mass) of an algal body was collected by centrifugation of the third day of culture. To the algal body, 2 mL of pure water per g of wet mass of the algal body was added, and suspended. The total volume of the suspension was about 210 mL. In a 50 mL crushing tube, 10 mL of the suspension and 25 g of zirconia beads (diameter: 0.1 mm) were added, and crushing was carried out (2500 rpm for a minute) using Multi Beads Shocker (Yasui Kikai Corporation) to obtain homogenized algal material.

### [Water extraction of homogenized algal material]

The homogenized algal material was gelled using a gelling agent.

The gelled homogenized algal material was added to an extraction tank with pure water in a volume 71. 4 times larger than the wet mass (wet weight) of the algal body used for crushing, and water extraction was carried out with stirring using a stirrer under room temperature (about 25°C) for 24 hours. During water extraction, pure water (extraction solvent) was supplied into the extraction tank at a rate of 41 mL/min.

Citric acid was added to the liquid discharged from the extraction tank to make a concentration of 0.0064%(w/v), and the obtained liquid was used for subsequent column purification.

### [Column purification of water extract]

A column having 150 mL of a packed adsorbent material (product name: SP850, supplier: Mitsubishi Chemical Corporation) was used (inner diameter 3 cm x height 22.5 cm).

The whole amount of the liquid discharged from the extraction tank was loaded onto the column, and subsequently 450 mL of pure water was applied thereto over 15 minutes (first washing of the adsorbent material).

The adsorbent material taken out of the column was moved to a 1 L bottle with 450 mL of pure water, and the obtained mixture was stirred with a stirrer for 10 minutes to remove solid material mixed in the adsorbent material (second washing).

A 40% aqueous solution of ethanol (450 mL) was added to the washed adsorbent material, and the obtained mixture was stirred with a stirrer for 10 minutes to remove foreign substances from the adsorbent material (third washing).

The adsorbent material was packed in a column (inner diameter 5 cm x height 50 cm) again and 450 mL of 100 % ethanol was applied thereto. The effluent was collected every 50 mL and 9 fractions (50 mL x 9) was taken.

The forth to eighth fractions in the order of elution were mixed and the obtained mixture was subjected to an evaporator to obtain brown solid material.

To the solid material, 3 mL of ethanol was added to obtain an ethanol solution.

The ethanol solution was subjected to centrifugation to remove ethanol-insoluble components.

The supernatant obtained by centrifugation was cooled at -30°C for 30 minutes and further subjected to centrifugation to remove solid material.

The obtained supernatant was cooled again at -30°C for 30 minutes and subjected to centrifugation.

The obtained supernatant was used as a Nannochloropsis water extract for a componential analysis and an animal test described below.

### [Componential analysis of Nannochloropsis water extract]

A componential analysis was carried out using thin layer chromatography (TLC) and liquid chromatography-mass spectrometer (LC-MS).

The Nannochloropsis water extract was spotted on a silica gel TLC plate and developed with two developing solvents. The developing solvents were 100% methanol (mobile phase 1) and a mixed solvent of hexane, diethyl ether, and acetic acid (volume ratio = 20 : 80 : 2) (mobile phase 2) .

Three spots A to C which appeared on the TLC using the mobile phase 1 and eight spots D to K which appeared on the TLC using the mobile phase 2 were scraped off from the TLC plate. Each was extracted again using 1 mL of ethanol and subjected to LC-MS analysis in the following conditions.

### HPLC conditions

| | |
|---|---|
| Device | Nexera-I LC-2040C 3D |
| Column | KinetexTM C8 (2.1 mm ID × 150 mm L, 2.6 µm) |
| Column oven | 40°C |
| Solvent A | 0.1% formic acid-water |
| Solvent B | Acetonitrile |
| Flow rate | 0.4 mL |
| Injection amount | 10 µL |

### MS conditions

| | |
|---|---|
| Device | LCMS-8050 |
| Ionization method | ESI positive/negative |
| Mode | MRM |

The following compounds were identified from the spots B, C, E, and F.

| Spots | Identified compounds |
|---|---|
| B | A compound of the general formula (I) (wherein R₁ and R₂ represent combination A or B, and HEPE is 8-HEPE, 11-HEPE, 15-HEPE, or 18-HEPE). |
| | A compound of the general formula (I) (wherein R₁ and R₂ represent combination C or D, and HETE is 11-HEPE or 15-HETE). |
| | A compound of the general formula (I) (wherein R₁ and R₂ represent combination E or F) . |
| C | A compound of the general formula (II) (wherein R₁ and R₂ represent combination A or B, and HEPE is 8-HEPE, 11-HEPE, 15-HEPE, or 18-HEPE). |
| | A compound of the general formula (II) (wherein R₁ and R₂ represent combination C or D, and HpEPE is 8-HpEPE, 11-HpEPE, 15-HpEPE, or 18-HpEPE). |
| E | 15-HEPE |
| F | 13-HOTrE |
| | 13-HODE |
| | 15-HETE |
| | 15-HETrE |

### [Animal test]

The effects of the Nannochloropsis water extract on (1) body weight increase and (2) cholesterol synthesis promotion were evaluated in laboratory animals having a high-fat diet.

### [Test animal]

C57 BL/6N male mice (Charles River Japan, Inc.) introduced at 6-week age were acclimated for a week and then used.

### [Diet]

A low-fat diet (LFD) (containing 10% of fat in an energy ratio) and a high-fat diet (HFD) (containing 45% of fat in terms of an energy ratio) were used. Lard was used as fat, and the weight adjustment of the amount of fat contained was carried out by a carbohydrate. The amounts of nutrients other than the fat and carbohydrate were designed to be equivalent per unit energy. Both feed and water were taken ad libitum.

### [Administration test sample]

Administration test samples containing the Nannochloropsis water extract were prepared according to the following procedure.

The Nannochloropsis water extract was diluted with ethanol so that the amount of 15-HEPE contained was 0.2 mg/mL (used to prepare low-dose samples) or 3.6 mg/mL (used to prepare high-dose samples), and the diluted solution was then divided into vial containers in a daily amount used. The obtained containers were stored in a medical freezer (-20°C) until the day of usage.

The Nannochloropsis water extract was moved to an animal testing laboratory on ice (aluminum block) and left to stand at room temperature under light shielding from 30 minutes before preparing the samples.

The Nannochloropsis water extract was diluted 20-fold with ultrapure water and then subjected to inversion mixing 10 times. The extract was then left to stand at room temperature under light shielding for 15 minutes. Administration test samples containing the Nannochloropsis water extract at a low dose and a high dose described below were obtained. It should be noted that the concentration of ethanol in each sample was 5%(v/v).

| | Amount of 15-HEPE (mg/mL) |
|---|---|
| Low-dose sample (HEPE Low dose; HL) | 0.01 |
| High-dose sample (HEPE High dose; HH) | 0.18 |

### [Animal test group]

Evaluation was made in 4 test groups described below.

| | |
|---|---|
| Test group 1 (n = 8) | LFD intake + solvent (5% (v/v) ethanol) administration (LFD) |
| Test group 2 (n = 9) | HFD intake + solvent (5% (v/v) ethanol) administration (HFD) |
| Test group 3 (n = 8) | HFD intake + low-dose administration (HFD + HL) |
| Test group 4 (n = 8) | HFD intake + high-dose administration (HFD + HH) |

The test group 1 was set as a low-fat diet control group. A 5% (v/v) aqueous ethanol solution, corresponding to a solvent used to prepare the administration test samples, was administered.

The test group 2 was set as a high-fat diet control group. A 5% (v/v) aqueous ethanol solution, corresponding to a solvent used to prepare the administration test samples, was administered.

The test group 3 was set as a high-fat diet + low-dose administration group. The amount of 15-HEPE administered in the test group 3 was 0.1 mg/kg body weight/day.

The test group 4 was set as a high-fat diet + high-dose administration group. The amount of 15-HEPE administered in the test group 4 was 1.8 mg/kg body weight/day.

### [Administration method]

A test sample was administered to a test animal under restrained conditions by an orogastric tube.

The low-dose sample and the high-dose sample were each administered in an amount of 100 µL per 10 g of mouse body weight.

Once daily administration was continued for 21 days in each of the test groups 1 to 4. Administration was carried out between 8 am to 12 noon on each day.

### [Management of laboratory animals]

The animals were reared in the following environment.

### [Rearing facility]

Temperature: 22 ± 1.5°C
Humidity: 45 ± 5%
Light and dark cycle: light period 8:00 to 20:00, dark period 20:00 to 8:00

### Individual rearing

### [Example 1: Effect of suppressing body weight increase]

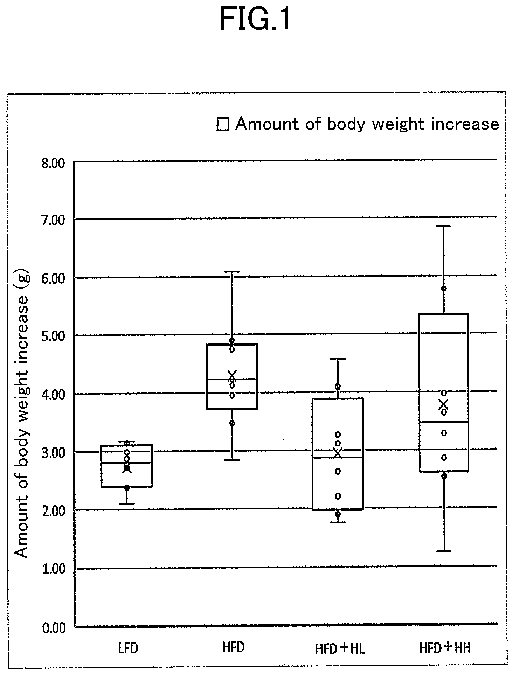

The "amount of body weight increase from the base date" was obtained from the measurement values of body weight on the first day of the experiment (base date) and the 22nd day of the experiment, the next day of the final administration day. Fig. 1 shows the distribution of the amount of body weight increase in individuals of each test group.

The amount of body weight increase from the first day of the experiment (base date) to the 22nd day of the experiment, the next day of the final administration day, was assessed according to Steel's multiple comparison test. The results are shown in Table 1-1 and 1-2.

**[Table 1-1]**

| Test statistic | | |
|---|---|---|
| Test group 2 vs Test group 1 | Test group 2 vs Test group 3 | Test group 2 vs Test group 4 |
| 3.0792 | 2.4056 | 1.1547 |

**[Table 1-2]**

| p-value | | |
|---|---|---|
| Test group 2 vs Test group 1 | Test group 2 vs Test group 3 | Test group 2 vs Test group 4 |
| 0.00592 | 0.04369 | 0.52317 |

A significant difference (p < 0.05) was observed between the test group 1 (LFD) and the test group 2 (HFD) at a significance level of 0.05. The results show that body weight increased by high-fat diet (HFD) intake compared to low-fat diet (LFD) intake.

A significant difference (p < 0.05) was observed between the test group 2 (HFD) and the test group 3 (HFD + HL). The results show that body weight increase associated with high-fat diet intake was suppressed by administering a low-dose Nannochloropsis water extract (HL).

A significant difference was not observed between the test group 2 (HFD) and the test group 4 (HFD + HH); however, the administration of a high-dose Nannochloropsis water extract (HH) is also thought to be able to suppress body weight increase associated with high-fat diet intake by dividing daily intake into multiple times (so that the amount of the algal water extract taken per once will be nearly equal to that of HL).

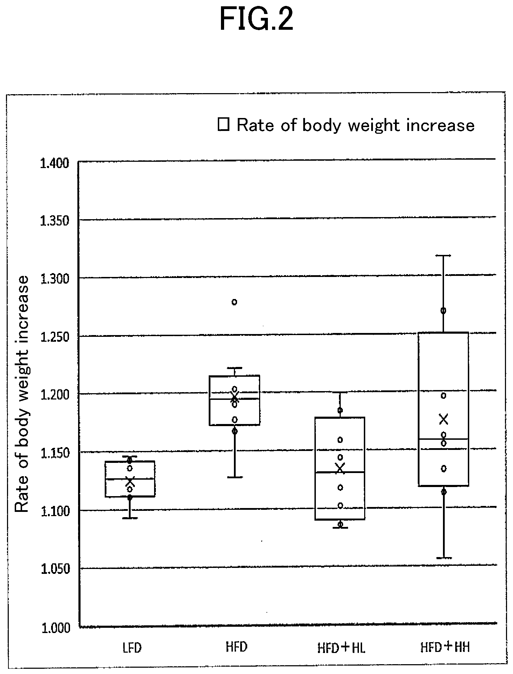

The "rate of body weight increase from the base date" was obtained from the measurement values of body weight on the first day of the experiment (base date) and the 22nd day of the experiment, the next day of the final administration day. Fig. 2 shows the distribution of the rate of body weight increase in individuals of each test group.

The rate of body weight increase from the first day of the experiment (base date) to the 22nd day of the experiment, the next day of the final administration day, was assessed according to Steel's multiple comparison test. The results are shown in Table 2-1 and 2-2.

**[Table 2-1]**

| Test statistic | | |
|---|---|---|
| Test group 2 vs Test group 1 | Test group 2 vs Test group 3 | Test group 2 vs Test group 4 |
| 3.0792 | 2.5019 | 1.0585 |

**[Table 2-2]**

| p-value | | |
|---|---|---|
| Test group 2 vs Test group 1 | Test group 2 vs Test group 3 | Test group 2 vs Test group 4 |
| 0.00595 | 0.03381 | 0.59009 |

A significant difference (p < 0.05) was observed between the test group 1 (LFD) and the test group 2 (HFD) at a significance level of 0.05. The results show that body weight increased by high-fat diet (HFD) intake compared to low-fat diet (LFD) intake.

A significant difference (p < 0.05) was observed between the test group 2 (HFD) and the test group 3 (HFD + HL). The results show that body weight increase associated with high-fat diet intake was suppressed by administering a low-dose Nannochloropsis water extract (HL).

A significant difference was not observed between the test group 2 (HFD) and the test group 4 (HFD + HH); however, the administration of a high-dose Nannochloropsis water extract (HH) is also thought to be able to suppress body weight increase associated with high-fat diet intake by dividing daily intake into multiple times (so that the amount of the algal water extract taken per once will be nearly equal to that of HL).

### [Example 2: Effect of inhibiting cholesterol synthesis promotion]

Liver tissue collected from the test animals on the 22nd day of the experiment, the next day of the final administration day, was subjected to a transcriptome analysis using DNA microarray (Thermo Fisher Scientific, Clariom S array, Mouse). The variation of expression of genes involved in the cholesterol synthesis system was analyzed between the test groups according to the following conditions.

### Combination of comparison between the groups:

Test group 2 (HFD) vs Test group 1 (LFD),
Test group 2 (HFD) vs Test group 3 (HFD + HL),
Test group 2 (HFD) vs Test group 4 (HFD + HH).
Computation environment: R version 4.0.3.
Normalization: qFARMS (FARMS information: https://pubmed.ncbi.nlm.nih.gov/16473874/).
Between-group comparison analysis: Rank Products 2 (Rank Products 2 information: https://pubmed.ncbi.nlm.nih.gov/28481966/).

As an index of remarkable expression variation, "false discovery rate (FDR)" was used, and 11 genes meeting the following "variation condition 1" and "variation condition 2-1 or 2-2" were judged as a "gene involved in cholesterol synthesis promotion, the expression of which is increased in association with high-fat diet intake (condition 1) but is reduced (inhibited) by administering a Nannochloropsis water extract (condition 2)" (Table 3).
Variation condition 1:
   FDR < 0.05 in Test group 2 (HFD) > Test group 1 (LFD)
Variation condition 2-1:
   FDR < 0.05 in Test group 2 (HFD) > Test group 3 (HFD + HL)
Variation condition 2-2:
   FDR < 0.05 in Test group 2 (HFD) > Test group 4 (HFD + HH).

**[Table 3]**

| Gene name | ENTREZ _ID | UNIGEN E ID | REFSEQ ID | FDR | | |
|---|---|---|---|---|---|---|
| | | | | Test group 2 (HFD) > Test group 1 (LFD) | Test group 2 (HFD) > Test group 3 (HFD+HL) | Test group 2 (HFD) > Test group 4 (HFD+HH) |
| 3-hydroxy-3-methylglutaryl-Coenzyme A synthase 1 | 208715 | Mm.615 26 | NM_001291 439 | 0.0000001 391 | 0.0000127 958 | 0.0055727 869 |
| 3-hydroxy-3-methylglutaryl-Coenzyme A reductase | 15357 | Mm.316 652 | NM_008255 | 0.0000025 271 | 0.0001873 604 | 0.0051844 614 |
| Mevalonate (diphospho) decarboxylase | 192156 | Mm.281 46 | NM_138656 | 0.0006789 928 | 0.0017107 103 | 0.0167613 518 |
| Farnesyl diphosphate farnesyl transferase 1 | 14137 | Mm.474 432 | NM_010191 | 0.0000000 285 | 0.0001829 057 | 0.0090032 118 |
| Squalene epoxidase | 20775 | Mm.296 169 | NM_009270 | 0.0000047 930 | 0.0000377 920 | 0.0132658 995 |
| Lanosterol synthase | 16987 | Mm.550 75 | NM_146006 | 0.0003805 250 | 0.0003140 134 | 0.0769378 248 |
| Cytochrome P450, family 51 | 13121 | Mm.140 158 | NM_020010 | 0.0000002 907 | 0.0013525 378 | 0.0066505 20 |
| Methylsterol monoxygenas e 1 | 66234 | Mm.3011 9 | NM_025436 | 0.0000000 007 | 0.0007383 094 | 0.0396354 943 |
| NAD(P) dependent steroid dehydrogenas e-like | 18194 | Mm.387 92 | NM_010941 | 0.0000026 360 | 0.0002922 162 | 0.0055303 204 |
| Hydroxysteroi d (17-beta) dehydrogenas e 7 | 15490 | Mm.128 82 | NM_010476 | 0.0000090 541 | 0.0012164 458 | 0.1608499 257 |
| 7-dehydrocholes terol reductase | 13360 | Mm.249 342 | NM_007856 | 0.0005079 411 | 0.0007245 420 | 0.0103605 382 |

### INDUSTRIAL APPLICABILITY

The present invention can be used for e.g. food/drink and medicine.

## Claims

1. A body weight increase suppressing agent, comprising a water extract of Nannochloropsis algae as an active ingredient.

2. The suppressing agent according to claim 1, wherein the water extract comprises one or more compounds selected from [A group] described below:
[A group]
15-HEPE,
a monoacylglyceryltrimethylhomoserine represented by the general formula (I): 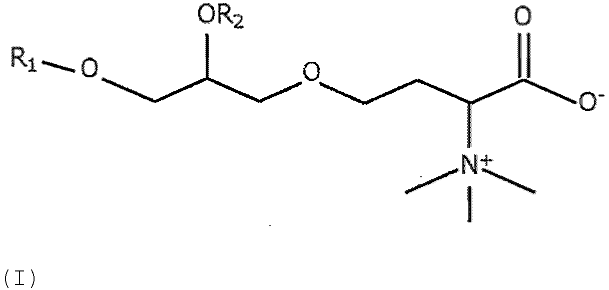 wherein R₁ and R₂ represent combination A, B, E, or F described below:
| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |
and
a lysophosphatidylcholine represented by the general formula (II) : 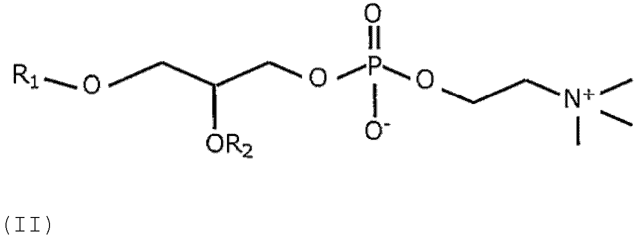 wherein R₁ and R₂ represent combination A or B described below.
| Combination | R₁ | R₂ |
|---|---|---|
| A | HEPE residue | Hydrogen |
| B | Hydrogen | HEPE residue |

3. The suppressing agent according to claim 2, wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:
| Combination | R₁ | R₂ |
|---|---|---|
| C | Hydroxy eicosatetraenoic acid (HETE) residue | Hydrogen |
| D | Hydrogen | HETE residue |
and
a lysophosphatidylcholine represented by the general formula (II),
wherein R₁ and R₂ represent combination C or D.
| Combination | R₁ | R₂ |
|---|---|---|
| C | Hydroperoxy eicosapentaenoic acid (HpEPE) residue | Hydrogen |
| D | Hydrogen | HpEPE residue |

4. The suppressing agent according to claim 1, wherein the Nannochloropsis algae are Nannochloropsis oceanica.

5. The suppressing agent according to claim 1, which suppresses body weight increase associated with high-fat diet intake.

6. Food/drink for suppressing body weight increase, comprising a water extract of Nannochloropsis algae as an active ingredient.

7. The food/drink according to claim 6, wherein the water extract comprises one or more compounds selected from [A group] described below:
[A group]
15-HEPE,
a monoacylglyceryltrimethylhomoserine represented by the general formula (I): 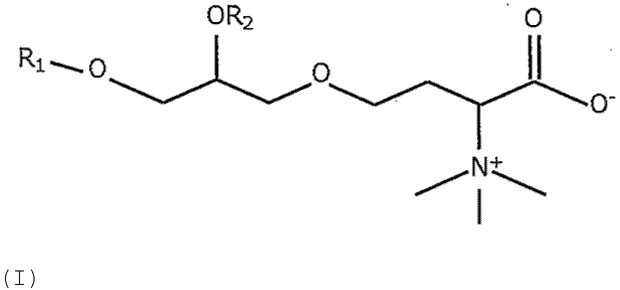 wherein R₁ and R₂ represent combination A, B, E, or F described below:
| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |
and
a lysophosphatidylcholine represented by the general formula (II): 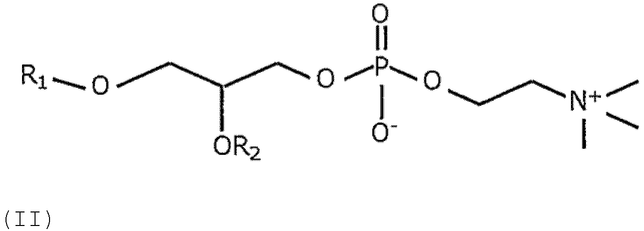 wherein R₁ and R₂ represent combination A or B described below.
| Combination | R₁ | R₂ |
|---|---|---|
| A | HEPE residue | Hydrogen |
| B | Hydrogen | HEPE residue |

8. The food/drink according to claim 7, wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:
| Combination | R₁ | R₂ |
|---|---|---|
| C | Hydroxy eicosatetraenoic acid (HETE) residue | Hydrogen |
| D | Hydrogen | HETE residue |
and
a lysophosphatidylcholine represented by the general formula (II),
wherein R₁ and R₂ represent combination C or D.
| Combination | R₁ | R₂ |
|---|---|---|
| C | Hydroperoxy eicosapentaenoic acid (HpEPE) residue | Hydrogen |
| D | Hydrogen | HpEPE residue |

9. The food/drink according to claim 6, wherein the Nannochloropsis algae are Nannochloropsis oceanica.

10. The food/drink according to claim 6, which suppresses body weight increase associated with high-fat diet intake.

11. A cholesterol synthesis promotion inhibitor comprising a water extract of Nannochloropsis algae as an active ingredient.

12. The inhibitor according to claim 11, wherein the water extract comprises one or more compounds selected from [A group] described below:
[A group]
15-HEPE,
a monoacylglyceryltrimethylhomoserine represented by the general formula (I): 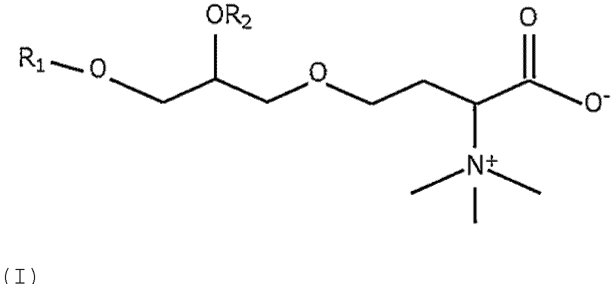 wherein R₁ and R₂ represent combination A, B, E, or F described below:
| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |
and
a lysophosphatidylcholine represented by the general formula (II) : 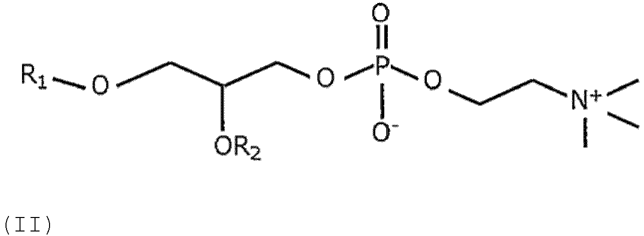 wherein R₁ and R₂ represent combination A or B described below.
| Combination | R₁ | R₂ |
|---|---|---|
| A | HEPE residue | Hydrogen |
| B | Hydrogen | HEPE residue |

13. The inhibitor according to claim 12, wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:
| Combination | R₁ | R₂ |
|---|---|---|
| C | Hydroxy eicosatetraenoic acid (HETE) residue | Hydrogen |
| D | Hydrogen | HETE residue |
and
a lysophosphatidylcholine represented by the general formula (II),
wherein R₁ and R₂ represent combination C or D.
| Combination | R₁ | R₂ |
|---|---|---|
| C | Hydroperoxy eicosapentaenoic acid (HpEPE) residue | Hydrogen |
| D | Hydrogen | HpEPE residue |

14. The inhibitor according to claim 11, wherein the Nannochloropsis algae are Nannochloropsis oceanica.

15. The inhibitor according to claim 11, which inhibits cholesterol synthesis promotion associated with high-fat diet intake.

16. Food/drink for inhibiting cholesterol synthesis promotion, comprising a water extract of Nannochloropsis algae as an active ingredient.

17. The food/drink according to claim 16, wherein the water extract comprises one or more compounds selected from [A group] described below:
[A group]
15-HEPE,
a monoacylglyceryltrimethylhomoserine represented by the general formula (I): 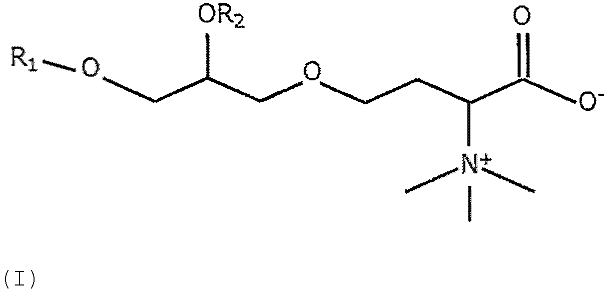 wherein R₁ and R₂ represent combination A, B, E or F described below:
| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |
and
a lysophosphatidylcholine represented by the general formula (II) : 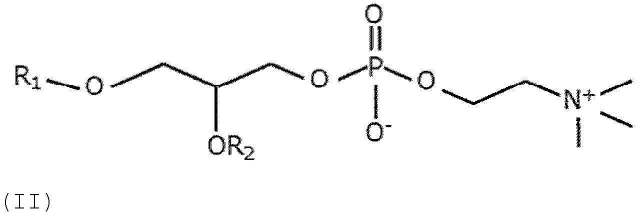 wherein R₁ and R₂ represent combination A or B described below.
| Combination | R₁ | R₂ |
|---|---|---|
| A | HEPE residue | Hydrogen |
| B | Hydrogen | HEPE residue |

18. The food/drink according to claim 17, wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:
| Combination | R₁ | R₂ |
|---|---|---|
| C | Hydroxy eicosatetraenoic acid (HETE) residue | Hydrogen |
| D | Hydrogen | HETE residue |
and
a lysophosphatidylcholine represented by the general formula (II),
wherein R₁ and R₂ represent combination C or D.
| Combination | R₁ | R₂ |
|---|---|---|
| C | Hydroperoxy eicosapentaenoic acid (HpEPE) residue | Hydrogen |
| D | Hydrogen | HpEPE residue |

19. The food/drink according to claim 16, wherein the Nannochloropsis algae are Nannochloropsis oceanica.

20. The food/drink according to claim 16, which inhibits cholesterol synthesis promotion associated with high-fat diet intake.

21. A pharmaceutical composition for treating a disease associated with cholesterol synthesis promotion, comprising a water extract of Nannochloropsis algae as an active ingredient.

22. The pharmaceutical composition according to claim 21, wherein the water extract comprises one or more compounds selected from [A group] described below:
[A group]
15-HEPE,
a monoacylglyceryltrimethylhomoserine represented by the general formula (I): 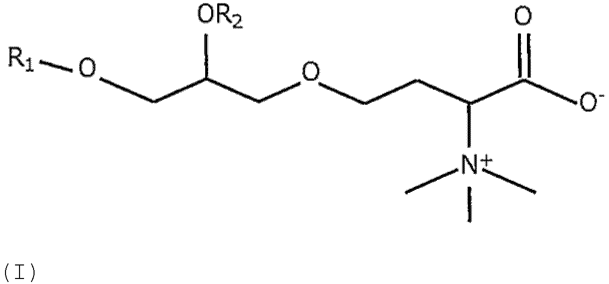 wherein R₁ and R₂ represent combination A, B, E, or F described below:
| Combination | R₁ | R₂ |
|---|---|---|
| A | Hydroxy eicosapentaenoic acid (HEPE) residue | Hydrogen |
| B | Hydrogen | HEPE residue |
| E | Eicosapentaenoic acid (EPA) residue | Hydrogen |
| F | Hydrogen | EPA residue |
and
a lysophosphatidylcholine represented by the general formula (II) : 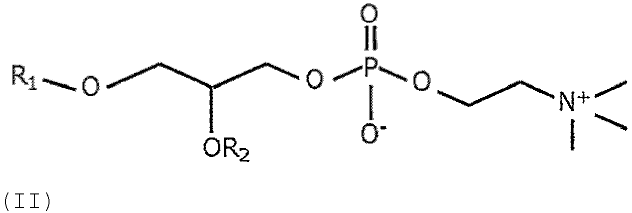 wherein R₁ and R₂ represent combination A or B described below.
| Combination | R₁ | R₂ |
|---|---|---|
| A | HEPE residue | Hydrogen |
| B | Hydrogen | HEPE residue |

23. The pharmaceutical composition according to claim 22, wherein the water extract further comprises one or more compounds selected from [B group] described below:
[B group]
13-HOTrE,
13-HODE,
15-HETrE,
15-HETE,
a monoacylglyceryltrimethylhomoserine represented by the general formula (I),
wherein R₁ and R₂ represent combination C or D described below:
| Combination | R₁ | R₂ |
|---|---|---|
| C | Hydroxy eicosatetraenoic acid (HETE) residue | Hydrogen |
| D | Hydrogen | HETE residue |
and
a lysophosphatidylcholine represented by the general formula (II),
wherein R₁ and R₂ represent combination C or D.
| Combination | R₁ | R₂ |
|---|---|---|
| C | Hydroperoxy eicosapentaenoic acid (HpEPE) residue | Hydrogen |
| D | Hydrogen | HpEPE residue |

24. The pharmaceutical composition according to claim 21, wherein the Nannochloropsis algae are Nannochloropsis oceanica.

25. The pharmaceutical composition according to claim 21, wherein the disease associated with cholesterol synthesis promotion is selected from a group consisting of non-alcoholic fatty liver disease (NAFLD), non-alcoholic steatohepatitis (NASH), dyslipidemia, and metabolic syndrome.
